# EUROPÄISCHE PATENTANMELDUNG

(11) **EP 4 792 791 A1**
(43) Veröffentlichungstag der Anmeldung: **19.08.2026**
(21) Anmeldenummer: 26158337.1
(22) Anmeldetag: 12.02.2026
(51) Int. Cl.: A61F 13/01

(54) **ABSORPTIONSVERBAND MIT INTEGRIERTER BINDE UND VERFAHREN ZUR HERSTELLUNG EINES ABSORPTIONSVERBANDES MIT INTEGRIERTER BINDE**

(30) Priorität: 13.02.2025 PL 45122325
(71) Anmelder: Politechnika Lodzka, 90-924 Lodz (PL); Tricomed Spolka Akcyjna, 93-493 Lodz (PL); Bella Spolka z ograniczona odpowiedzialnoscia, 87-100 Torun (PL); Wojskowy Instytut Higieny i Epidemiologii im. Gen. Karola Kaczkowskiego, 01-163 Warszawa (PL)
(72) Erfinder: KOLESINSKA, Beata, 95-575 Lodz (PL); MIKOLAJCZYK, Zbigniew, 94-262 Lodz (PL); KLONOWSKA, Magdalena, 94-011 Lodz (PL); PIEKLAK, Katarzyna, 91-496 Lodz (PL); DRACZYNSKI, Zbigniew, 95-073 Ustronie (PL); PAUL, Paulina, 93-173 Lodz (PL); LATANSKA, Ilona, 94-104 Lodz (PL); ROSIAK, Piotr, 91-863 Lodz (PL); SUJKA, Witold, 95-080 Tuszyn (PL); KOSTKOWSKI, Dariusz, 87-100 Torun (PL); BRYTAN, Marek, 05-071 Sulejowek (PL)
(74) Vertreter: Bischof, Oliver

(57) **Zusammenfassung**

Gegenstand der Erfindung ist ein Absorptionsverband (100) mit integrierter Binde (16), umfassend ein absorbierendes, nicht resorbierbares Wundkissen (1), welches aus saugfähigen Vliesstoff-Lagen und einer dampfdurchlässigen Folie besteht. Das Wundkissen ist in die Binde integriert. Die Binde enthält mindestens ein Klettverschluss-Element (9, 10). An einem ersten Ende (19) der Binde ist eine Hilfstasche (5) in Form eines beidseitig offenen Tunnels mit einer Breite von mindestens 5 cm angeordnet. In einem Abstand von mindestens 3 cm von der Hilfstasche (5) befindet sich eine Hülle (6) von einer Breite von mindestens 1 cm. Ein Druckelement (7) ist in die Hülle (6) eingesetzt, wobei die Öffnungen der Hülle (6) über eine Strecke von mindestens 2 mm verschlossen sind, um ein Herausrutschen des Druckelements zu verhindern. Die Hülle mit dem Druckelement (7) ist an der dem Wundkissen gegenüberliegenden Seite des Absorptionsverbands angenäht. An einem gegenüberliegenden Ende der Binde ist ein Tourniquet (8) angeordnet. Die Erfindung stellt außerdem ein Verfahren zur Herstellung dieses Absorptionsverbands bereit.

## Beschreibung

Gegenstand der Erfindung ist ein Absorptionsverband, der in eine Binde integriert ist, aufweisend ein absorbierendes, nicht resorbierbares, in die Binde integriertes Wundkissen, welches sich aus saugfähigen Vliesstoff-Schichten und einer dampfdurchlässigen Folienschicht zusammensetzt.

Auf dem Markt sind verschiedene absorbierende Wundauflagen mit hoher Absorptionskapazität erhältlich. Diese Wundauflagen werden für den militärischen Einsatz hergestellt, um Soldaten und Ersthelfern im Gefecht eine schnelle und optimale Wundversorgung zu ermöglichen. Sie bestehen aus mehreren Komponenten und dienen der Blutstillung und dem Schutz vor Infektionen. Allerdings weisen einige Wundauflagen zu wenig Absorptionsmaterial auf, sind unwirksam oder aufwendig in der Herstellung.

Das Patent US7652190 beschreibt einen textilen Träger für Wundverbände, der ein Wundheilungsmaterial enthält. Der textile Träger weist mindestens ein Befestigungselement auf der Verbandoberfläche auf, das ein unkontrolliertes Abwickeln des Verbandes verhindert. Das Befestigungselement besteht aus beabstandeten Streifen mit Klettverschluss oder Klebstoff.

Die Patentanmeldung US2007/0260165 A1 offenbart einen mit einer Binde integrierten Wundverband, dessen Wund-Kontaktierungsfläche eine Schichtstruktur aufweist. An der Binde sind gleichmäßig über ihre Gesamtlänge mehrere Klettverschluss-Elemente verteilt. Die an den Enden der Binde angeordneten Klettverschluss-Elemente erstrecken sich breiter als die übrigen Klettverschluss-Elemente.

Die bisher bekannten, in den obigen Dokumenten beschriebenen Konstruktionen weisen jedoch den Nachteil auf, dass die Klettverschlüsse in einem separaten Schritt des Herstellungsprozesses an der Binde befestigt oder geklebt werden, was zu einer lokalen Veränderung der Steifigkeit der Bandage führt, was ein versehentliches Abwickeln der Bandage während des Gebrauchs zur Folge haben kann.

Mit der vorliegenden Erfindung soll ein Absorptionsverband mit integrierter Binde zur Erstversorgung und umfassenden Behandlung von traumatischen Wunden entwickelt werden, bei dem ein unerwünschtes, versehentliches Abwickeln der Binde während des Gebrauchs vermieden werden kann.

Erfindungsgemäß umfassen die Verbandsstrukturen mindestens ein Klettverschluss-Element, welches in einem einzigen Herstellungsprozess der Binde in ihre Gewebestruktur, wie Strickgewebestruktur, vorzugsweise gleichmäßig über die gesamte Verbandslänge eingearbeitet wird. Von großem Vorteil ist, dass die Steifigkeit des Strickgewebes nicht lokal verändert und ein versehentliches Aufribbeln des Absorptionsverbands während des Gebrauchs verhindert werden kann. Das bzw. die Klettverschluss-Elemente sind entweder an den Rändern oder - vorzugsweise - im mittleren Bereich der Binde angeordnet.

Der Absorptionsverband ist für die schnelle und einfache Anwendung durch professionelle Rettungssanitäter und Laien konzipiert und gewährleistet so eine effektive, umfassende Behandlung, insbesondere von mäßig bis stark blutenden Wunden, auch an schwer zugänglichen Stellen wie Hals, Kopf, Achselhöhle und Leiste.

Der erfindungsgemäße Absorptionsverband kann Bestandteil militärischer Sanitätsausrüstung, ziviler medizinischer Ausrüstung und Krankenhausausstattung sein.

Im Weiteren werden folgende Begriffe mit ihren Pendanten verwendet:
"Absorptionsverband" = Wundverband, Verband
"Binde" = Bandage
"Wundkissen" = Polster
" Tourniquet" = Klemmelement
"Schicht" = Lage (v. Material)
" absorbierendes"= saugfähiges

Der erfindungsgemäß integrierte Absorptionsverband besteht aus einem nicht resorbierbaren, aus saugfähigem Vliesstoff gefertigten Polster, auch Wundkissen genannt und einer dampfdurchlässigen Folie. Der Polster liegt in direktem Kontakt mit der Wunde und ist in eine innovative elastische Binde integriert, deren Struktur ein Verdrehen verhindert. Die gleichmäßig innerhalb der Bindestruktur verteilten Klettverschluss-Elemente reduzieren zudem das Risiko einer Verbandkontamination und stabilisieren den Absorptionsverband nach der endgültigen Festlegung am Körper des Patienten.

An einem ersten Ende der Binde ist eine elastische Hilfstasche vorgesehen, welche das Anlegen des Absorptionsverbandes erleichtert und die einhändige Anwendung ermöglicht. Die Hilfstasche ist durchgängig, wodurch die Hand über eine oder zweite Taschenöffnung hineingesteckt werden und das Anlegen des Wundkissens an der Wunde beschleunigen kann. In das Innere der Hilfstasche kann ein zusätzliches Wundschutz-Material, wie Gaze und/oder Schutzfolie, platziert werden.

Die im Inneren der Hilfstasche untergebrachte Gaze gilt als zusätzliche Wundauflage und übt Druck genau dort aus, wo er benötigt wird. Eine Schutzfolie dient beispielsweise zur Sicherung von Brustwunden, als Okklusivverband bei Schusswunden und als Wärmeisolierung. Zwei Kunststoffkomponenten sind ebenfalls integraler Bestandteil des Produkts, nämlich ein schwenkbares Druckelement, das das Anlegen des Absorptionsverbandes erleichtert, indem es unter anderem die Verbandrichtung ändern kann und durch den Absorptionsverband zusätzlichen lokalen Druck auf die Wunde ausübt, sowie ein Tourniquet, das das Verband-Ende nach dem Anlegen fixiert.

Der textile Träger gemäß Erfindung stellt einen Verband in Form von elastischen Bändern dar, die mittels 3D-Kettenwirktechnik hergestellt werden. Der textile Träger besteht aus natürlichen Rohstoffen, z. B. Baumwolle oder baumwollähnlichen Materialien, oder aus synthetischen Rohstoffen wie Viskose, Polyamid, Polypropylen und Elastomere.

Darüber hinaus ist der textile Träger mit Säurefarbstoff behandelt, der seine schlechte Sichtbarkeit im Infrarotbereich, insbesondere im nahen Infrarot, gewährleistet, was für verwundete Soldaten bei Nachtbedingungen von großer Bedeutung ist.

Die noch nicht geformte Hilfstasche kann nahtlos in einem einzigen Arbeitsschritt zusammen mit der Binde gefertigt werden. Erst danach kann das freie Ende noch nicht fertigen Hilfstasche umwickelt und an der Binde angenäht werden. Die Hilfstasche bildet dann einen beidseitig offenen Tunnel mit einer Breite von 5 bis 15 cm.

In einem Abstand 5 - 10 cm von der Hilfstasche entfernt kann eine 1 bis 10 cm breite Hülle, die das schwenkbare Druckelement aufnimmt, an der Binde befestigt, vorzugsweise angenäht werden. Die Öffnungen der Hülle sind 3 bis 15 mm weit verschlossen, um ein Herausrutschen des dort eingesetzten Druckelements zu verhindern. Die Hülse des Druckelements ist auf der dem nicht resorbierbaren Polster gegenüberliegenden Seite des Absorptionsverbandes angeordnet.

Auf einem der Hilfstasche gegenüber liegenden Ende des Absorptionsverbandes ist ein an sich bekanntes Klemmelement, wie Tourniquet vorgesehen.

Die Hilfstasche ist nicht nur für das geschickte Manipulieren mit dem Absorptionsverband vorgesehen. In deren Innere können unterschiedliche herausnehmbare, für die Wundheilung geeignete Utensilien und zusätzliche saugfähige Verbandmaterialien, beispielsweise Gaze, gelegt werden. Die saugfähigen Verbandmaterialien können aus Polyester, Polyamid, Polypropylen, Polyurethan, Polyethylen, Polyacrylnitril, Zellulose oder Viskose bestehen. Die wichtigsten Parameter für die Verwendung eines bestimmten Vliesstoffs bzw. Absorptionsbandes sind Flächenmasse, Absorptionskapazität und Reißfestigkeit.

Bei der für den Absorptionsverband verwendeten Gaze handelt es sich um einen 5 bis 20 cm breiten und 1 bis 10 m langen Vliesstoffstreifen, der in Lagen gefaltet oder zu einer Rolle aufgerollt und in die besagte Hilfstasche gelegt wird.

Die Schutzfolie, beispielsweise zum Schutz von Brustwunden, kann aus Polyethylen oder Polyurethan bestehen. Für den erfindungsgemäßen Absorptionsverband kann eine Folie mit einer beispielhaften Oberfläche von 100 - 900 cm² zum Einsatz kommen, die in Lagen mehrfach gefaltet in die vorgenannte Hilfstasche herausnehmbar untergebracht wird.

Der erfindungsgemäße Absorptionsverband besteht aus einem nicht resorbierbaren Wundkissen aus mehreren Lagen eines saugfähigen Vliesstoffs und einer dampfdurchlässigen Folie.

Vorzugsweise weist das Wundkissen vier Lagen auf, nämlich drei sandwichartig zueinander angeordnete Vliesstoff-Schichten und eine dampfdurchlässige Folienschicht, die mit den Vliesstoff-Schichten an den Rändern des Wundkissens miteinander vernäht ist. Dabei liegt zwischen einer inneren, mit der jeweiligen Wunde zu kontaktierenden Vliesstoff-Schicht und einer weiteren gleichen Vliesstoff-Schicht die dritte, flauschigere Vliesstoff-Schicht.

Alle Lagen des absorbierenden Vliesmaterials können aus Polyester, Polyamid, Polypropylen, Polyurethan, Polyethylen, Polyacrylnitril, Zellulose, Viskose oder anderen Materialien bestehen.

Die wichtigsten Parameter für die Verwendung des saugfähigen Vliesstoffs für einen Absorptionsverband sind Flächenmasse, Absorptionskapazität und Reißfestigkeit. Eine dampfdurchlässige, auslaufsichere Folie aus Polyurethan kann eine Dicke von weniger als 30 µm und eine Flächenmasse von weniger als 50 g/m² aufweisen.

Es ist von Vorteil, wenn das absorbierende Wundkissen dauerhaft in einem Abstand von 0 - 15 mm von der Hilfstasche entfernt mit der Binde vernäht ist und/oder als verschiebbarer Polster ausgeführt ist, welcher es ermöglicht, eine optimale oder gewünschte Position des Wundkissen entlang der Binde auszuwählen.

Vorteilhaft ist, wenn das Wundkissen nur an drei Seiten dauerhaft an die Binde genäht wird, wodurch eine zusätzliche Aufbewahrungstasche entsteht, in die ein zusätzliches absorbierendes Material eingelegt werden kann.

Das Druckelement und das Tourniquet bestehen aus einem für Medizinprodukte geeigneten Material. Zu den wichtigsten Konstruktionsparametern zählen die Größe der Kontaktfläche mit dem Absorptionsverband und das Gewicht des Materials.

Ein integraler Bestandteil des Absorptionsverbandes ist das vorzugsweise aus Kunststoff gefertigte, schwenkbare Druckelement in der Form einer Gürtelschnalle, deren Steg innerhalb der mit der Binde verbundenen Hülle geführt ist und um deren Bügel ein Teil der Binde geführt und nach Richtungsänderung angespannt werden kann, wodurch auf die Wunde ein zusätzlicher Druck ausgeübt wird.

Das Tourniquet ist ebenso ein fester Bestandteil des Absorptionsverbandes. Es wird mithilfe eines Textilträgers am Ende des Verbandes angenäht und verhindert nach dem Anlegen ein Abwickeln, indem die Zinken des Tourniquets in eine der Lagen der bereits gewickelten Binde eingehakt werden.

Ein Verfahren gemäß Erfindung zur Herstellung des Absorptionsverbandes, der in eine Binde integriert ist und einen saugfähigen, nicht resorbierbaren Polster von einer Schichtstruktur enthält, aufweisend Vliesstoff-Lagen und einen dampfdurchlässigen Folienabschnitt ist dadurch gekennzeichnet, dass mindestens ein Klettverschluss-Element in die Materialstruktur der Binde integriert ist.

Die Klettverschluss-Elemente werden während eines einzigen Herstellungsvorgangs der Binde in die Strickstruktur eingearbeitet, vorzugsweise gleichmäßig über die gesamte Länge der Binde verteilt. Das oder die Klettverschluss-Elemente werden an den Längsrändern der Binde oder/und vorzugsweise in ihrer Mittelteil durchgehend oder als diskontinuierlicher Streifen eingebracht.

Die Bandage (Binde) weist an ihrem ersten Ende die Hilfstasche in Form eines 5 -15 cm breiten, beidseitig offenen Tunnels.

5 - 10 cm von der Hilfstasche entfernt wird eine 1 bis10 cm breite Hülle angenäht, deren Länge nahezu über die ganze Breite, d.h. senkrecht zu ihrer Längsausrichtung, reicht. In die Hülle wird ein Steg des klammerartigen Druckelement eingesetzt. Die offenen Ränder der Hülse sind jeweils über eine Länge von 3-15 mm verschlossen, um ein Herausrutschen des Druckelementes zu verhindern. Die das Druckelement haltende Hülle wird auf der dem nicht resorbierbaren Wundkissen gegenüberliegenden Seite an die Binde genäht.

An dem der Hilfstasche gegenüberliegenden Ende der Binde wird ein Tourniquet angelegt.

Die Binde bzw. der ganze Absorptionsverband ist mit einem Säurefarbstoff beschichtet, wodurch er unter Infrarotlicht einen geringen Kontrast zur Umgebung aufweist. Die Gaze und Schutzfolie werden in zusammengefalteter Form in die Hilfstasche eingeführt. Vorteilhaft ist zudem das Einrichten einer verschiebbaren Verbandlasche, in die das Wundkissen eingelegt wird. So kann unterhalb des Wundkissens eine Aufbewahrungstasche gebildet werden, in der ein zusätzliches saugfähiges Material platziert wird.

Die erfindungsgemäße Verbandstruktur verhindert zudem ein Verdrehen und Abwickeln des Wundverbands. Der Wundverband weist eine heterogene, dreidimensionale Struktur mit Elementen auf, die ein Abwickeln dank Klettverschluss verhindern. Die Strickstruktur des Wundverbandes besteht aus mindestens vier Nadelkämmen, von denen mindestens drei die Basisstruktur bilden. Ein vierter Nadelkamm oder mehrere Nadelkämme führen Monofilamentgarn in die Textilstruktur ein und bilden so die sogenannte Schlaufenabdeckung des Klettverschlusses. Die entwickelte Verbandstruktur ist heterogen, da die Schlaufenabdeckung (Klettverschluss) durchgehend oder diskontinuierlich an den Rändern oder im Mittelteil des Wundverbandes verläuft. Die Höhe der Monofilament-Schlaufenabdeckung wird nicht nur durch die Webart, sondern auch durch den Abstand zwischen den Nadelkämmen bestimmt.

Der Verband, dessen Struktur ein Verdrehen und Abwickeln verhindert, wurde auf einer Doppelspindel-Kettwirkmaschine mit E18-Teilung unter Verwendung der 3D-Doppelkettwirktechnologie hergestellt. Die Grundstruktur des Verbandes basiert vorteilhafterweise auf drei Komponentensträngen, wobei ein viertes Komponent eine dreidimensionale Schlaufenstruktur erzeugt, die nach dem Schneiden der Schlaufen und der Weiterverarbeitung die Struktur des Klettverschluss-Elements bildet.

Die Schlaufen bestehen aus neun 0,1 mm dicken Polypropylen-Monofilamentfäden, die in die Mitte des Gewebes eingefädelt werden. Sie ragen 5-10 mm über die Gewebeoberfläche hinaus. Die Schlaufen werden auf zwei Nadelkämmen erzeugt. Die restlichen drei Strickgarne bestehen aus Polyamidgarn (560 dtex) und Polyurethangarn (223/1 dtex). Das Klettgewebe selbst entsteht durch das Einfädeln der Monofilamentfäden auf den gegenüberliegenden Nadelkamm und das Abwerfen der entstandenen Schlaufen. Im nächsten Verarbeitungsschritt werden die Schlaufenenden mit einer Schere abgeschnitten und die synthetischen Fäden auf eine Höhe von 1-5 mm gekürzt.

Im Gegensatz zu geflochtenen Fäden nehmen Monofilamente keine Flüssigkeiten und Bakterien auf, daher eignen sich besonders gut für die Behandlung von infektionsgefährdeten Wunden.

Gegenstand der Erfindung ist in zwei Ausführungsbeispielen anhand der Zeichnung dargestellt. Die Figuren zeigen: :
- Fig. 1: Position der Hilfstasche und der Hülle für das Druckelement,
- Fig. 2: Anordnungen der Klettverschluss-Elemente an der Binde,
- Fig. 3: Anordnungen der Gaze und Schutzfolie innerhalb des Tunnels, bei einer zusammengerollten Binde,
- Fig. 4: Aufbau des saugfähigen Wundverbandes (Wundkissens) in einer perspektivischen Darstellung,
- Fig. 5: Anordnungen des saugfähigen Wundkissens an der Binde,
- Fig. 6: Anordnung des Druckelementes und des Tourniquets als integraler Teilen der Binde, in einer Draufsicht auf Außenfläche der Binde,
- Fig. 7: schematischen Aufbau des mit der Binde integrierten Absorptionsverbands, in einer Draufsicht auf die Innenseite sowie auf die Außenseite der aufgerollten Binde,
- Fig. 8: ein aus Kunststoff geformtes Druckelement in mehreren Aussichten,
- Fig. 9: ein Tourniquet für die Binde, perspektivisch und in einer Seitenansicht.

### Beispiel 1

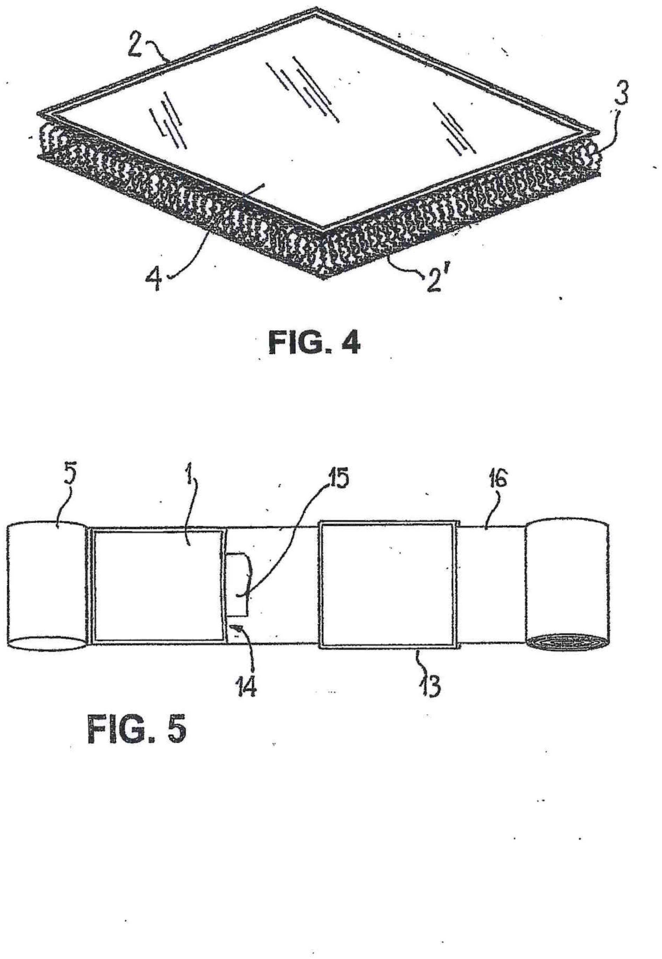

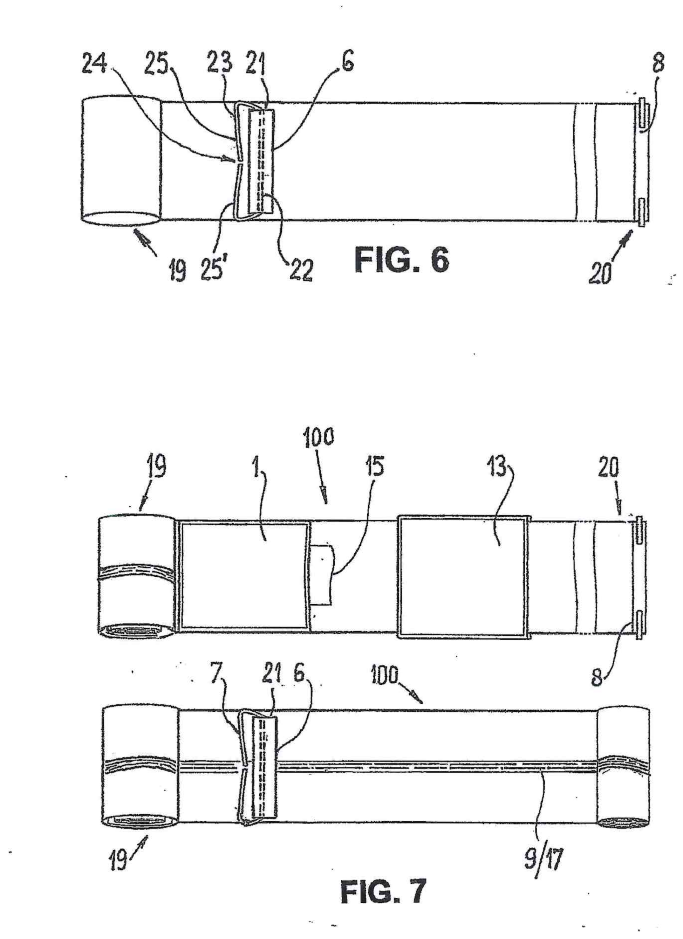

Der mit der Binde 16 integrierte Absorptionsverband 100 (vgl. Fig. 7) besteht aus einem saugfähigen, nicht resorbierbaren, viereckigen Wundkissen 1 in Sandwich-Anordnung, das zwei voneinander beabstandete Vliesstoff-Abschnitte 2, 2', einen dazwischen liegenden weiteren, in Fig. 4 gezeigten deutlich flauschigeren Vliesstoff-Abschnitt 3 und einen dampfdurchlässigen Folienabschnitt 4 umfasst, sowie aus einer mit dem Wundkissen 1 integrierten Binde 16. Die Binde 16 weist eine Materialstruktur auf, die ein versehentliches Abwickeln der besagten Binde verhindert.

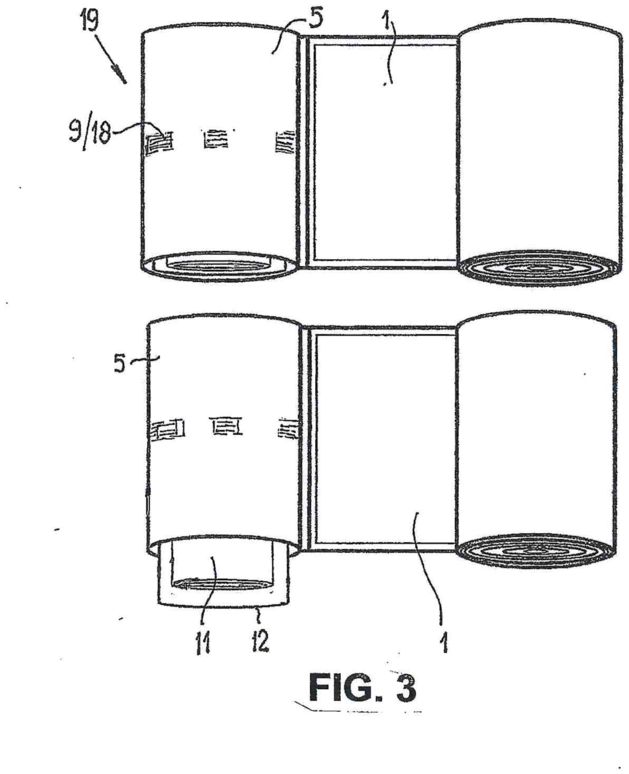

Auf einem ersten Ende 19 ("Anfang") der Binde 16 ist eine Hilfstasche 5 in Form eines beidseitig offenen Tunnels mit einer Breite von 5 bis 15 cm vorgesehen. Ins Innere der Hilfstasche 5 können gemäß Fig. 3 zusammengefalteten zusätzlichen Verbandmaterialien, wie Gaze 11 und Schutzfolie 12 eingeführt sein. Die Fig. 3 (obere Abbildung) stellt die zusammengerollte Binde 16 mit in den Tunnel gelegten Gaze 11 und Schutzfolie 12 dar, wobei die untere Abbildung der Figur zeigt diese zusätzlichen Verbandmaterialien während der Herausnahme.

In einem Abstand von 5 bis 10 cm von der Hilfstasche 5 befindet sich eine 1 bis 10 cm breite Hülle 6, in der das Druckelement 7 platziert ist. Die Öffnungen der Hülle 6 sind über einen Abschnitt von 3 - 15 mm geschlossen, um zu verhindern, dass das Druckelement 7 herausrutscht. Die Hülle 6 ist auf der dem nicht resorbierbaren Wundkissen 1 gegenüberliegenden Seite der Binde 16 an dieser genäht.

Wie in Figuren 6 und 7 dargestellt, ist das Druckelement 7 etwa in Form einer Gürtelschnalle, im vorliegenden Fall aus Polyamid, ausgeführt. Das schwenkbare Druckelement 7 sitzt mit seinem Fuß 22 in der Hülle 6, dagegen sein Bügel 23 ragt über die Hülle 6 hinaus. Der Bügel 23 weist zwei Arme 25, 25' auf, deren freie Enden eine Öffnung 24 (Abstand) bilden, die es ermöglicht, den Fuß 22 in die Hülle 6 einzuführen. Außerdem ist der Bügel 23 etwa konkav bzw. bogenförmig, mit dem Bogenscheitel auf die Hülle 6 zeigend ausgeführt, so dass die Arme 25, 25' beim Anspannen der Binde 16 während ihrer Richtungsänderung nachgeben können.

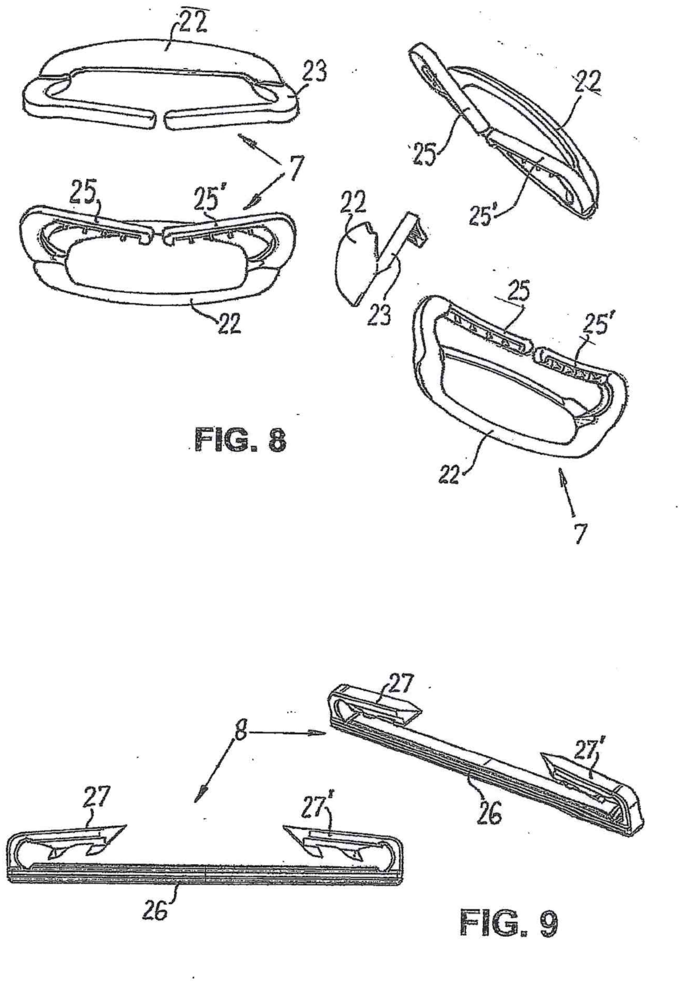

Der Fig. 8 ist eine andere Ausführungsform des Druckelementes 7 dargestellt, das sich mit gleichen Bezugszahlen, wie das Druckelement gemäß Figuren 6 ,7 beschreiben lässt. Der Konstruktionsunterschied der beiden Ausführungsformen besteht darin, dass das Druckelement 7 gemäß Figuren 6 und 7 einer Schnalle aus Draht ähnelt, dagegen das Druckelement 7 gemäß Fig. 8 ein Formstück aus Kunststoff (Polyamid) mit gezahnten Armen 25, 25' und breiteren Fuß 22 ist. Die gezahnten Arme 25, 25' bewirken, dass die durch eine solche Klemmschnalle geführte Binde 16 blitzschnell festgeklemmt werden kann, was von großem Vorteil bei der Wundbehandlung von verwundeten Soldaten ist.

An einem dem ersten Ende 19 gegenüber liegenden zweiten Ende 20 der Bandage ist ein Klemmelement, wie Tourniquet 8, vorgesehen, welches detailliert in Fig. 9 dargestellt ist. Das über einen textilen Träger an der Binde eingesetzte Tourniquet 8 weist eine Basis 26 und zwei spiegelsymmetrisch aufeinander gerichtete, gezahnte Arme 27, 27' auf.

Die Binde ist mit einem tarnenden Farbstoff, wie Säurefarbstoff behandelt, der die Sichtbarkeit im Infrarotbereich verschlechtert.

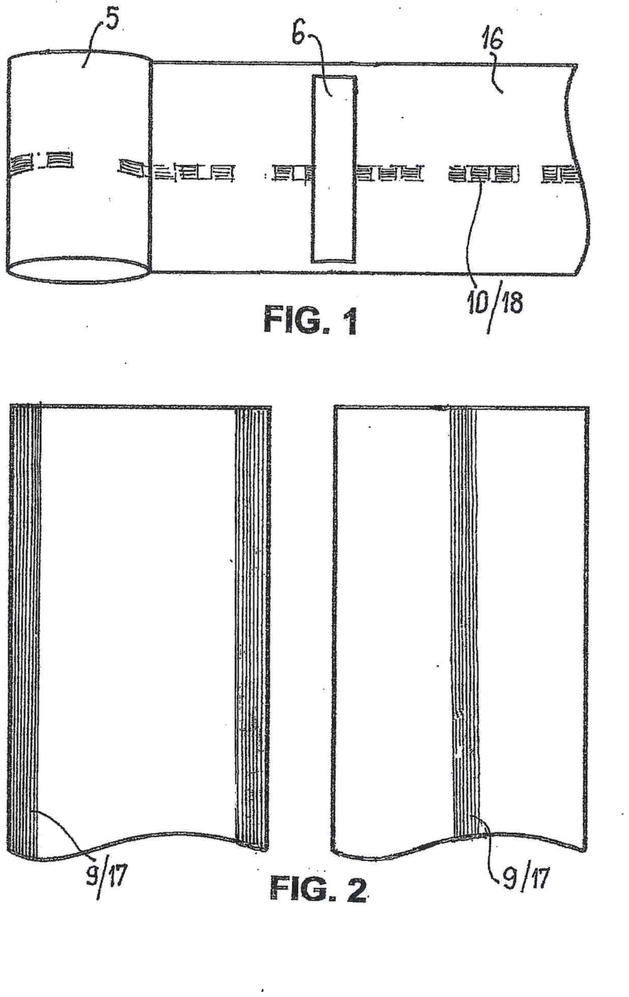

Der Absorptionsverband 100 zeichnet sich durch eine heterogene, räumliche Struktur mit mindestens einem Klettverschluss-Element 9,10 (vgl. Figuren 1 und 2) auf. Die Klettverschluss-Elemente 9,10 befinden sich entweder an den Längsrändern des Verbandes (vgl. Fig. 2, linke Abbildung) oder im Mittelteil des Verbandes und bilden durchgehende Streifen 17 aus. Optional bilden die Klettverschluss-Elemente voneinander beabstandete Streifen-Abschnitte 18 aus, wie der Fig. 1 zu entnehmen ist.

Die Klettverschluss-Elemente 17, 18 bestehen aus synthetischen Monofilamentfäden mit einer Dicke von 0,5 - 1,5 mm, wobei die Höhe der synthetischen Monofilamentfäden von 1 - 5 mm beträgt. Die Monofilamentfäden sind mit einer Verdickung versehen.

Gemäß den Figuren 5 und 7 (obere Abbildung) ist an der Binde 16 optional eine entlang der Binde verschiebbare Verbandlasche 13 angeordnet, in der das Wundkissen 1 untergebracht und positioniert werden kann. Weiterhin kann das Wundkissen 1, falls an seinen drei Kanten mit der Binde 16 zusammengenäht ist, eine Aufbewahrungstasche 14 für das weitere zusätzliche Verbandmaterial 15 bilden.

### Beispiel 2

Das Verfahren zur Herstellung eines erfindungsgemäßen Absorptionsverbands 100 mit integrierter Bandage 16 beruht auf folgenden Arbeitsschritten:
- Einbringen der Klettverschluss-Elemente 9, 10 in die Materialstruktur der Binde 16,
- Anfertigung einer Hilfstasche 5 durch Umwickeln eines freien Ende der Binde 16 und Verbinden mit dem Material der Binde unter Bildung eines durchgehenden Tunnels,
- Schneiden der Halbprodukte, wie Vliesstoff-Bänder, in zwei Vliesstoff-Schichten 2, 2' und eine dritte, deutlich flauschigere Vliesstoff-Schicht 3,
- Bereitstellung eines flüssigkeitsdichten, dampfdurchlässigen Folienabschnitts 4 von derselben Ausmaßen, wie die der Vliesstoff-Schichten 2, 2',
- Zusammenlegen der Vliesstoff-Schichten 2, 2', 3 und des Folienabschnitts 4 zu einem rechteckigen Wundkissen 1, indem die dickere, flauschigere Vliesstoff-Schicht 3 zwischen die beiden Vliesstoff-Schichten 2, 2' platziert und der dampfdurchlässige Folienabschnitt 4 auf die eine Vliesstoff-Schicht 2 gelegt wird; Zusammennähen ihrer Ränder,
- Anbringen einer Hülle 6 an der Binde 16 in einem Abstand von 5-10 cm von der Hilfstasche 5 und Einsetzen eines Druckelements 7 in die Hülle 6 ,
- Verschließen der Öffnungen 21 der Hülle 6 über eine Strecke von 3-15 mm zum Verhindern des Herausrutschens des Druckelements 7 aus der Hülle 6,
- Hineinlegen der Gaze 11 und Schutzfolie 12 (als zusätzlicher Verbandstoff) in die Hilfstasche 5,
- Behandeln der Binde 16 mit einem tarnenden Mittel, wie Säurefarbstoff,
- Einsetzen eines Tourniquets 8 an dem der Hilfstasche 5 gegenüber liegenden Ende der Binde.
- Optionales Einsetzen einer verschiebbaren Verbandlasche 13 zur Unterbringung des Wundkissens,
- Optionale Ausbildung einer Aufbewahrungstasche 14 unter dem Wundkissen 1 für ein weiteres Verbandmaterial 15.

### Bezugszeichenliste:

- 1: saugfähiges Wundkissen
- 2, 2': Vliesstoff- Schicht (v. 1)
- 3: Vliesstoff-Schicht (v. 1)
- 4: Folienabschnitt
- 5: Hilfstasche
- 6: Hülle
- 7: Druckelement /Klammer
- 8: Tourniquet
- 9, 10: Klettverschluss-Elemente
- 11: Gaze
- 12: Schutzfolie
- 13: Verbandlasche
- 14: Aufbewahrungstasche
- 15: Verbandmaterial (zusätzlich)
- 16: Binde
- 17: Streifen durchgehend (v. 9,10)
- 18: Streifenabschnitte (v. 9,10)
- 19: Ende (v.16)
- 20: Ende (v.16)
- 21: Öffnung (v. 6)
- 22: Fuß (v. 7)
- 23: Bügel (v. 7)
- 24: Öffnung/Abstand
- 25, 25': Arm (v. 23)
- 26: Basis (v. 8)
- 27, 27': gezahnter Arm
- **100**: **Absorptionsverband**

## Patentansprüche

1. Absorptionsverband (100) mit integrierter Binde (16), aufweisend ein absorbierendes, nicht resorbierbares, in die Binde (16) integriertes Wundkissen (1), welches sich aus saugfähigen Vliesstoff-Schichten und einer dampfdurchlässigen Folienschicht zusammensetzt,
**dadurch gekennzeichnet, dass**
- in die Materialstruktur der Binde (16) mindestens ein Klettverschluss-Element (9, 10) eingebaut ist,
- wobei die Binde (16) auf ihrem einen Ende (19) in eine Hilfstasche (5) ausläuft und auf dem gegenüber liegenden Ende (20) ein Tourniquet (8) trägt,
- wobei die Hilfstasche (5) als beidseitig offener Tunnel von einer Innenbreite 5 bis 15 cm ausgeführt ist,
- wobei in einem Abstand 5 bis 10 cm von der Hilfstasche (5) eine 1 bis 10 cm breite Hülle (6) auf der dem nicht resorbierbaren Wundkissen (1) gegenüberliegenden Seite der Binde (16) angenäht ist, in der ein schwenkbares Klemmelement (7) platziert ist,
- wobei die Hülle (6) an ihren beiden Öffnungen (21) über eine Länge von 3-15 mm verschlossen ist, um ein Herausrutschen des Klemmelements (7) zu verhindern,
- wobei das Klemmelement (7) auf der dem nicht resorbierbaren Wundkissen (1) gegenüber liegenden Seite der Binde angenäht ist.

2. Absorptionsverband (100) nach Anspruch 1, **dadurch gekennzeichnet, dass**
- das saugfähige Wundkissen (1) drei Vliesstoff-Schichten (2, 2'; 3) und eine dampfdurchlässige Folienschicht (4) aufweist,
- wobei die innere, mit der jeweiligen Wunde zu kontaktierende Vliesstoff-Schicht (2) und die weitere Vliesstoff-Schicht (2') die dritte, flauschigere Vliesstoff-Schicht (3) mit einschließen,
- und wobei die vierte, dampfdurchlässige Folienschicht (4) mit den besagten Vliesstoff-Schichten (2, 2'; 3) an den Rändern des saugfähigen Wundkissens ((1) miteinander vernäht sind.

3. Absorptionsverband (100) nach Anspruch 1 oder 2, **dadurch gekennzeichnet, dass** die Binde (16) mit einem Säurefarbstoff beschichtet ist, der einen geringen Kontrast der Binde zur Umgebung im Infrarotlicht bewirkt.

4. Absorptionsverband (100) nach einem der Ansprüche 1 bis 3, **dadurch gekennzeichnet, dass** die Klettverschluss-Elemente (9, 10) aus synthetischen Monofilamentfäden mit einer Dicke von 0,5 bis 1,5 mm bestehen, deren Höhe 15 mm beträgt und die jeweils in eine Verdickung auslaufen.

5. Absorptionsverband (100) nach einem der Ansprüche 1 bis 4, **dadurch gekennzeichnet, dass** die Klettverschluss-Elemente (9, 10) entlang der Ränder oder im Mittelteil der Binde (16), vorzugsweise über deren gesamte Länge, in Form von durchgehenden Streifen (17) oder voneinander beabstandeten Abschnitten (18) angeordnet sind.

6. Absorptionsverband (100) nach einem der Ansprüche 1 bis 5, **dadurch gekennzeichnet, dass** innerhalb der Hilfstasche (5) eine zusammengefaltete Gaze (11) und eine zusammengefaltete Schutzfolie (12) abnehmbar platziert sind.

7. Absorptionsverband (100) nach einem der Ansprüche 1 bis 6, **dadurch gekennzeichnet, dass** an der Binde (16) wenigstens eine, längst der Binde (16) verschiebbare Verbandlasche (13) zur Aufnahme des absorbierenden Wundkissens (1) angeordnet ist.

8. Absorptionsverband (100) nach einem der Ansprüche 1 bis 7, **dadurch gekennzeichnet, dass** unter dem absorbierenden Wundkissens (1) eine Aufbewahrungstasche (14) angeordnet ist, in die ein zusätzliches absorbierendes Material (15) einlegbar ist.

9. Verfahren zur Herstellung eines Absorptionsverbands (100) mit integrierter Binde (16), umfassend ein absorbierendes Material in Form eines nicht resorbierbaren, mehrlagigen Wundkissens (1), das sich aus saugfähigen Vliesstoff-Schichten und einer dampfdurchlässigen Folie zusammensetzt,
**dadurch gekennzeichnet, dass**
- mindestens ein Klettverschluss-Element (9, 10) in die Materialstruktur der Binde (16) eingearbeitet wird,
- an einem ersten Ende (19) der Binde (16) eine Hilfstasche (5) in Form eines beidseitig offenen Tunnels von einer Breite von 5-15 cm ausgebildet wird,
- in einem Abstand von 5-10 cm von der Hilfstasche (5) an der Binde (16) eine 1-10 cm breite Hülle (6) auf der dem nicht resorbierbaren Wundkissen (1) gegenüberliegenden Seite der Binde (16) angenäht wird,
- in die Hülle (6) ein schwenkbares Druckelement (7) eingeführt wird, wobei die Öffnungen (21) der Hülle (6) über eine Länge von 3-15 mm verschlossen werden, um ein Herausrutschen des Druckelements zu verhindern,
- und an dem der Hilfstasche (5) gegenüberliegenden Ende (20) der Binde (16) ein Tourniquet (8) eingesetzt wird.

10. Verfahren nach Anspruch 9, **dadurch gekennzeichnet, dass**
- die Klettverschluss-Elemente (9, 10) in Form von synthetischen Monofilamentfäden mit einer Dicke von 0,5 bis 1,5 mm in die Verbandstruktur eingebracht werden,
- wobei die Höhe der synthetischen Monofilamentfäden auf 1 bis 5 mm festgelegt wird,
- und anschließend die Enden der Monofilamentfäden verdickt werden.

11. Verfahren nach Anspruch 9 oder 10, **dadurch gekennzeichnet, dass** die Binde (16) mit einem tarnenden Farbstoff beschichtet ist, der die Sichtbarkeit des Absorptionsverbandes (100) unter Infrarotlicht einschränkt.

12. Verfahren nach einem der Ansprüche 9 bis 11, **dadurch gekennzeichnet, dass** die Klettverschluss-Elemente (9, 10) entlang der Ränder oder im Mittelteil der Binde (16), vorzugsweise über die gesamte Länge der Binde (16), durchgehend oder diskontinuierlich eingebracht werden.

13. Verfahren nach einem der Ansprüche 9 bis 12, **dadurch gekennzeichnet, dass** in die Hilfstasche (5) zusätzliche Verbandmaterialien, wie Gaze (11) und Schutzfolie (12) abnehmbar platziert werden.

14. Verfahren nach einem der Ansprüche 9 bis 13, **dadurch gekennzeichnet, dass** an der Binde (16) eine verschiebbare Verbandlasche (13) einsetzbar ist, in welche das absorbierende Wundkissen (1) untergebracht wird.

15. Verfahren nach einem der Ansprüche 9 bis 14, **dadurch gekennzeichnet, dass** unter dem absorbierenden Wundkissen (1) eine Aufbewahrungstasche (14) ausgebildet wird, in die das zusätzliche absorbierende Material (15) eingelegt werden kann.
